# EUROPÄISCHE PATENTSCHRIFT

(11) **EP 0 691 974 B1**
(45) Veröffentlichungstag und Bekanntmachung des Hinweises auf die Patenterteilung: **07.05.1997**
(21) Anmeldenummer: 94913090.0
(22) Anmeldetag: 30.03.1994
(51) Int. Cl.: C07F 7/12, C07F 7/18, C07B 37/02, C07J 1/00

(54) **ALKYLIERUNGSMITTEL UND VERFAHREN ZUR 1,4-ADDITION EINER ALKYLGRUPPE AN EINE ALPHA, BETA-UNGESÄTTIGTE KETO-VERBINDUNG**
ALKYLATING AGENT AND 1,4-ADDITION PROCESS OF AN ALKYL GROUP ONTO AN ALPHA, BETA-UNSATURATED KETONE COMPOUND
AGENT ALKYLANT ET PROCEDE D'ADDITION EN 1,4 D'UN GROUPE ALKYLE SUR UN COMPOSE CETONIQUE ALPHA, BETA-INSATURE

(30) Priorität: 30.03.1993 DE 4311028
(43) Veröffentlichungstag der Anmeldung: 17.01.1996
(73) Patentinhaber: SCHERING AKTIENGESELLSCHAFT, 13342 Berlin (DE)
(72) Erfinder: WESTERMANN, Jürgen, D-12161 Berlin (DE); NICKISCH, Klaus, D-12307 Berlin (DE)
(86) Internationale Anmeldenummer: EP9401001
(87) Internationale Veröffentlichungsnummer: WO9422878

(56) Entgegenhaltungen:
- EP-A- 0 534 582
- TETRAHEDRON LETTERS, Bd.21, 1980 Seiten 3389 - 3392 UTIMOTO, K. ET AL. 'CONJUGATED ADDITION OF CYANOTRIMETHYLSILANE TO .ALPHA.,.BETA.-UNSATURATED KETONES'
- TETRAHEDRON LETTERS, Bd.27, Nr.9, 1986 Seiten 1047 - 1050 ALEXAKIS, A. ET AL. 'ORGANOCOPPER CONJUGATE ADDITION REACTION IN THE PRESENCE OF TRIMETHYLCHLOROSILANE' in der Anmeldung erwähnt
- TETRAHEDRON LETTERS, Bd.27, Nr.34, 1986 Seiten 4029 - 4032 NAKAMURA, E. ET AL. 'ME3SICL ACCELERATED CONJUGATE ADDITION OF STOICHIOMETRIC ORGANOCOPPER REAGENTS' in der Anmeldung erwähnt

## Beschreibung

Die vorliegende Erfindung betrifft ein Alkylierungsmittel und ein Verfahren zur 1,4-Addition einer Alkylgruppe an ein α,β-ungesättigtes oder ein α,β-doppelt ungesättigtes Keton oder an einen α,β-ungesättigten Aldehyd.

Die 1-Methyleinführung an Steroiden ist ein erster und ein wichtiger Syntheseschritt bei der Herstellung von 1-Methylsteroiden. Beispiele dieser Substanzklasse sind Atamestan (1) (1-Methylandrosta-1,4-dien-3,17-dion), ein Inhibitor der Ostrogenbiosynthese (Aromatasehemmer) und Mesterolon (2)(1α-Methylandrosta-17β-ol-3-on), ein Steroid mit androgener Wirkung.

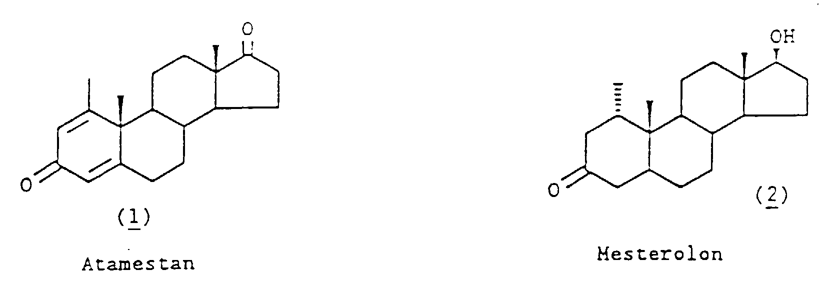

Eine bekannte Methode zur 1-Methyleinführung an z.B. (3) (Androsta-1,4-dien-3,17-dion) zu (4) (1α-Methylandrost-4-en-3,17-dion) ist die Addition von Dimethylkupfer-Lithium, welches aus Methyllithium und Kupfer-I-halogeniden hergestellt wird. Dazu sind molare Mengen des entsprechenden Kupfersalzes erforderlich. Zur Erzielung eines vollständigen Umsatzes bei der Umsetzung zu 4 ist ein deutlicher Überschuß an Reagenz Me₂CuLi erforderlich.

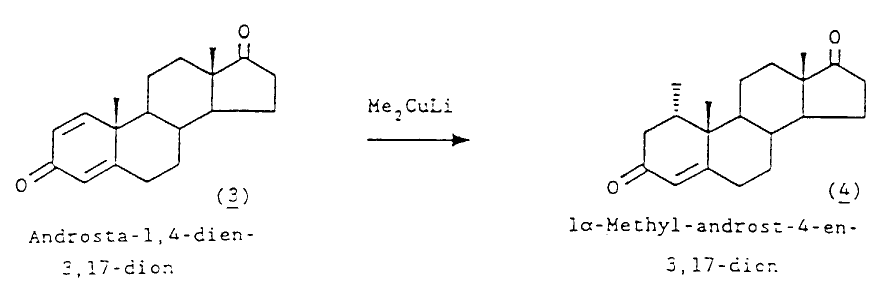

Dieses Verfahren ist Gegenstand der deutschen Patentschriften 2.046 640 und 2 253 087. Ein großes Problem stellen dabei die in molarer Menge anfallenden Kupfer-Salze dar, die aufgearbeitet werden müssen, sich aber durch Filtration nur äußerst schwierig abtrennen lassen.

Der Anfall größerer Mengen an Kupfer-Salzen läßt sich durch Kupfer-I-katalysierte 1,4-Addition mit Methylmagnesiumhalogeniden vermeiden, es findet dabei aber eine unerwünschte 1,2-Addition als Nebenreaktion statt. So läßt sich unter diesen Bedingungen Androsta-1,4-dien-3,17-dion (3) nicht zum gewünschten Produkt 1α-Methyl-androst-4-en-3,17-dion (4) methylieren. Vielmehr bildet sich hier durch Angriff an der 3-Carbonylgruppe und nach Wasserabspaltung aus dem intermediär gebildeten Carbinol 3-Exomethylen-androsta-1,4-dien-17-on (5).

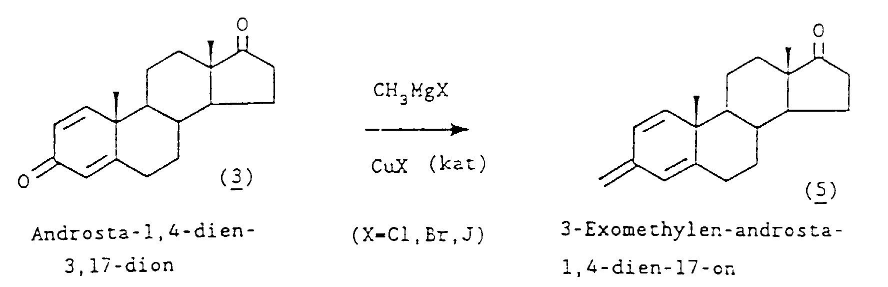

Es gibt in der Literatur nur wenige Beispiele einer 1,4-Addition an ein α,β-doppelt ungesättigtes Carbonylsystem, wie es im Beispiel des Androsta-1,4-dien-3,17-dions (3) der Fall ist. Um direkt aus 3 zu 4 zu gelangen, sind, wie oben dargelegt, stets molare Mengen an Dimethylkupfer-Lithium erforderlich, die aus molaren Mengen Methyllithium und Kupfer-I-halogeniden hergestellt werden müssen.

Als weitere Methode zur Einführung einer Methylgruppe in ein Steroid in 1-Position unter katalytischen Bedingungen ist die im nachfolgenden Schema gezeigte Reaktionsfolge von M. Tanabe und D.F. Crowe in Can. J. Chem. 45, 475 (1967) und in der deutschen Patentschrift 1 223 837 beschrieben.

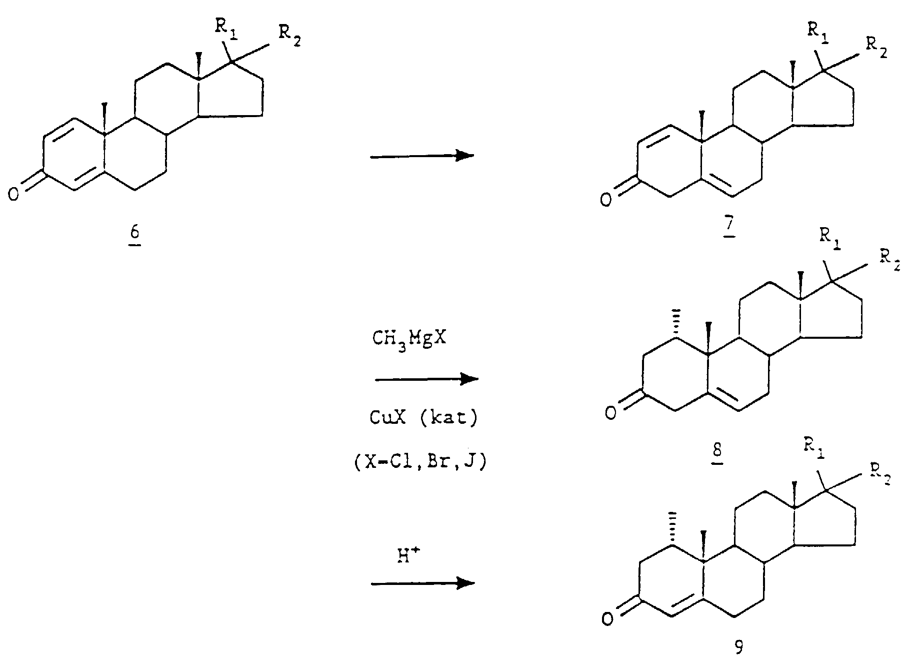

Dazu ist zunächst eine Überführung des 1,4-Dien-Systems 6 in eine 1,5-Dien-Steroidverbindung 7 erforderlich.

Die anschließende Addition an das dekonjugierte 1,5-Dien-System 7 ist unter katalytischen Bedingungen möglich, auch wenn die Ausbeuten bei dem entscheidenden Additionsschritt 7 zu 8 in der Praxis 50 % nicht überschreiten. Wegen zusätzlicher Stufenzahl und damit verbundener reduzierter Gesamtausbeute liegt mit der oben beschriebenen mehrstufigen Sequenz insgesamt betrachtet kein vorteilhaftes und ökonomisches Verfahren vor.

Es gibt also bisher für die Einführung einer Methylgruppe in 1-Position in ein 3-Keto-1,4-dien-Steroid (1,4-Addition), beispielsweise Androsta-1,4-dien-3,17-dion (3), kein brauchbares übergangsmetall-katalytisches Verfahren sowie kein geeignetes Methylierungsmittel.

In der nicht-vorveröffentlichten deutschen Patentanmeldung P 41 32 755.1 ( entspr. europäische Patentanmeldung Nr. 92250276.0, Veröffentlichungsnummer 0 534 582) wird ein neues Alkylierungsmittel, welches Trimethylaluminium oder Dimethylzink bzw. Triethylaluminium als Methyl- bzw. Ethylquelle sowie zusätzlich katalytische Mengen einer oder mehrerer Kupfer-I- und/oder Kupfer-II-Verbindungen enthält, sowie ein neues, übergangsmetall-katalysiertes Verfahren zur Addition einer Methyl- bzw. Ethylgruppe an ein α,β-ungesättigtes oder ein α,β-doppelt ungesättigtes Keton oder einen α,β-ungesättigten Aldehyd unter Verwendung dieses neuen Alkylierungsmittels beschrieben.

Vorzugsweise enthält das Alkylierungsmittel insgesamt 5 -10 Mol-% der Kupfer-I-und/oder Kupfer-II-Verbindung bezogen auf die zu alkylierende α,β-ungesättigte Ketoverbindung.

Als Kupfer-I- und/oder Kupfer-II-Verbindung(en) kommen in erster Linie eine oder mehrere Verbindung(en) der allgemeinen Formel I

CuX oder CuX₂ (I),

worin X einen einwertigen Rest darstellt und für Chlor, Brom, Iod, Cyano, den Thienyl-, Phenyl-, einen Alkoxy-, Thioalkoxy-, wobei der darin enthaltene Alkylrest 1 bis 8 Kohlenstoffatome besitzt und gegebenenfalls verzweigt und/oder ungesättigt ist, für einen substituierten Alkinylrest R-C≡C-, wobei R einen Phenyl- oder einen gegebenenfalls verzweigten C₁-C₈-Alkylrest bedeutet, oder für den Rest einer anorganischen Säure oder einer Carbonsäure oder für einen zweizähnigen, über Sauerstoff- und/oder Stickstoffatome koordinierenden Komplexliganden, ausgenommen den Liganden Acetylacetonat im Fall des zweiwertigen Kupfers, steht und/oder eine oder mehrere Verbindung(en) der allgemeinen Formel II

Cu₂Y oder CuY (II),

worin Y einen zweiwertigen Rest darstellt und für Sauerstoff oder Schwefel steht, infrage. Ist X der Rest einer anorganischen Säure, ist beispielsweise an den Hydrogencarbonat-, Hydrogensulfatrest oder an ähnliche Reste gedacht. Als Rest einer organischen Säure kommt vor allem der Acetat-Rest infrage.

Insbesondere ist an Kupfer-I- und/oder II-chlorid oder -bromid sowie Kupfer-I-cyanid als Übergangsmetall-Katalysator gedacht.

Aluminiumtrimethyl- und -ethyl sowie Zinkdimethyl können als Toluol- oder Hexan-Lösung eingesetzt werden. Wegen der problematischen Handhabung und der Selbstentzündlichkeit der Metallalkyle ist deren Verwendung in Lösung gegenüber der Verwendung in reiner Form vorzuziehen.

Aluminiumorganische Verbindungen gehen unter normalen Rektionsbedingungen ohne Zusatz eines Katalysators nur in geringem Umfang eine 1.2-Addition ein. Es ist literaturbekannt, daß erst unter drastischen Reaktionsbedingungen (höhere Temperaturen) eine solche Reaktion stattfindet. Unter normalen Bedingungen läuft eine solche Reaktion nur ab, wenn ein zweites Molekül Trimethylaluminium zur Verfügung steht. Das erste Molekül Trimethylaluminium komplexiert die Carbonylgruppe, an die so aktivierte Carbonylgruppe addiert dann das zweite Molekül [E.C. Ashby et al., J. Am.Chem. Soc., 90 (1968) 5179]. Eine Übersicht über Aluminiumalkyle findet man bei T. Mole und E.A. Jeffry in "Organoaluminium Compounds", Elsevier 1972, Seite 294 ff. 1,4-Additionen sind mit Me₂AIJ möglich, verlaufen aber in Konkurrenz zur 1,2-Addition und ohne Zusatz von Kupfer als Katalysator ab [J. Ashley et al., J. Org. Chem., 44 (1979)] und sind nicht selektiv.

Bei dem erwähnten Verfahren zur 1,4-Addition einer Methyl- bzw. Ethylgruppe an eine α,β-ungesättigte Ketoverbindung wird diese α,β-ungesättigte Ketoverbindung mit Aluminiumtrimethyl oder Zinkdimethyl bzw. Aluminiumtriethyl in Gegenwart einer katalytischen Menge einer oder mehrerer Kupfer-I- und/oder Kupfer-II-Verbindungen alkyliert.
Die gewünschte 1,4-Addition läuft glatt ab.

Für eine solche katalytische Verfahrensweise gibt es in der Literatur noch keine Beispiele.

Mechanistisch betrachtet wird bei dem erwähnten Verfahren eine Kupfer-I-Verbindung in eine Methyl-Kupfer bzw. Dimethylkupferverbindung überführt, die als solche die 1,4-Addition bewirkt. Nach Übertragung einer Methylgruppe auf das Substrat (Enon) kann das reaktive und 1,4-selektive Kupferreagenz in einem Kreisprozeß aus Trimethylaluminium erneut gebildet werden.

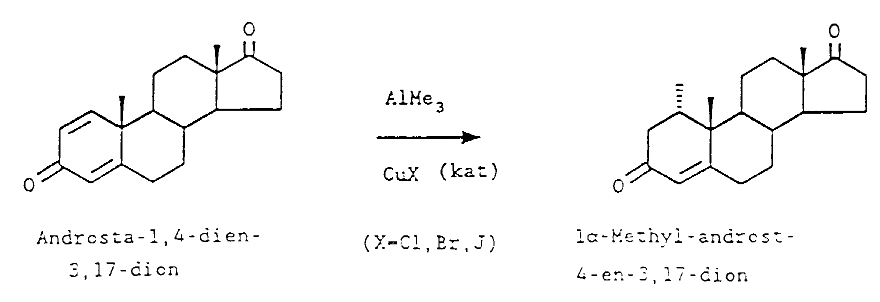

Als Kupfer-I-halogenid wird vorzugsweise das Chlorid oder Bromid eingesetzt. Die Reaktion wird vorzugsweise in Tetrahydrofuran, Dioxan, Dimethoxyethan, Toluol oder auch in Ethylacetat als Lösungsmittel durchgeführt. Überraschend dabei ist, daß unter den gefundenen Reaktionsbedingungen keine Reaktion mit der Carbonsäureestergruppe des Ethylacetates stattfindet. Ein Vorteil von Ethylacetat als Lösungsmittel liegt in der Umweltverträglichkeit dieses Solvens, das aus den natürlich vorkommenden Gruppen Essigsäure und Ethanol zusammengesetzt ist und in der Umwelt in diese natürlichen Moleküle hydrolysiert bzw. abgebaut werden kann.
Neben Kupfer-I-halogeniden sind auch Kupfer-II-halogenide, Kupfer-II-Verbindungen wie CuO und CuS für die Reaktion geeignet.
Als gut geeignet haben sich auch Kupfer-II-Komplexe erwiesen, in denen das Kupfer mit Liganden koordiniert ist.
Solche Komplexe der Formel 10 und 11 sind in der Literatur von L. Sacconi et al. in J. Chem. Soc., 1964, 276 beschrieben, die sich vom Salicylaldehyd ableiten und aus diesem herstellen lassen. 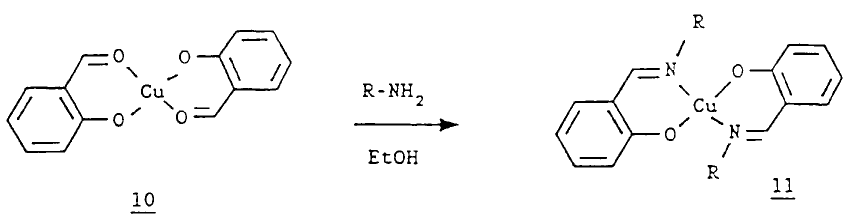
- R =: Alkyl: Methyl, Ethyl, n-Propyl, i-Propyl, n-Butyl, i-Butyl, tert.-Butyl, oder beide R zusammen stehen für die Gruppe -(CH₂)ₙ- (n = 2 oder 3)

Zugabe von Salicylaldehyd zu einer Kupfer-II-Salz-Lösung liefert nach Filtration den Komplex 10, der nach Isolierung und Umsetzung mit einem Amin wie z.B. Isopropylamin die Schiffsche Base im Komplex 11, worin R eine Isopropylgruppe bedeutet, liefert. Analog lassen sich weitere Komplexe herstellen.
Bei der Verwendung von Kupfer-II-Verbindungen als Katalysatoren stellen wahrscheinlich auch - durch Reduktion entstandene - Kupfer-I-Verbindungen die aktiven Spezies dar.

Bei all diesen Kupfer-I- und Kupfer-II-Verbindungen ist die Verwendung von katalytischen Anteilen ausreichend. Dabei wird vorzugsweise eine Menge von 5 - 10 Mol-% Kupferverbindung bezogen auf das eingesetzte Keton verwendet.

Erwähnenswert neben der ausgeprägten 1,4-Selektivität bei der Addition an ein 1,4-Dien-3-Ketosteroid ist die hohe 1-Selektivität dieser Addition. Es erfolgt bevorzugt eine Addition in 1-Position des Steroids zu 4, die deutlich gegenüber der sterisch stärker abgeschirmten 5-Position bevorzugt wird.
Das erwähnte Verfahren ist hoch stereoselektiv; der Anteil der als Nebenprodukt gebildeten 5β-Methylverbindung (im Fall der Methylierung von (3) 5β-Methyl-androst-1-en-3,17-dion) liegt unter 5% im Rohprodukt.
Ein weiterer Vorteil des beschriebenen Verfahrens ist, daß Acetylschutzgruppen unter den Reaktionsbedingungen erhalten bleiben. So gelingt beispielsweise die Überführung von 17β-Acetoxy-androst-1-en-3-on in 17β-Acetoxy-1α-methyl-5α-androstan-3-on mit einer Ausbeute von 89 %.
Die Isolierung der Produkte erfolgt vorzugsweise durch Kristallisation der Reaktionsprodukte oder durch Chromatographie. Die Ausbeuten an Produkt können bis zu 99% der Theorie betragen.

Die nach dem beschriebenen katalytischen Verfahren aus ADD (3) zugängliche Substanz 1α -Methylandrost-4-en-3,17-dion (4) ist eine wichtige Zwischenstufe zur Synthese von Mesterolon (2).

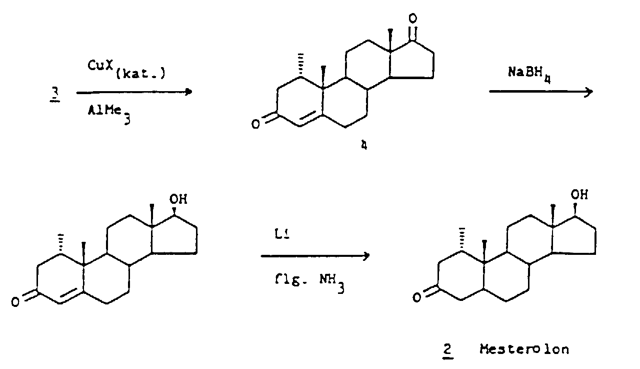

Literatur: DE-1 152 100 B; DE-2 046 640 B; NaBH₄-Reduktion: Fried & Edwards, Organic Reactions in Steroid Chemistry, Vol.I, 1972, S. 61 ff, Van Nostrand Reinhold Company, New York; Birch-Reduktion: Fried & Edwards, Vol. I, S. 39.
Auch 17β-Acetoxy-1α-methyl-5α-androstan-3-on ist als Ausgangsprodukt zur Herstellung von Mesterolon geeignet, welches sich aus ersterem bequem durch Verseifung der 17β-Acylgruppe erhalten läßt.

Wird ein Carbnsäureanhydrid oder -chlorid vor der Aufarbeitung der Reaktion 3 → 4 zu der Reaktionslösung gegeben, so kann das in der Reaktion vorliegende Enolat als Enolester z.B. 13 abgefangen werden.

Als Carbonsäureanhydrid oder -chlorid kommen die Anhydride der gerad- oder verzweigtkettigen Alkancarbonsäuren mit 2 bis 8 Kohlenstoffatomen, insbesondere der Essigsäure, sowie der Benzoesäure infrage.

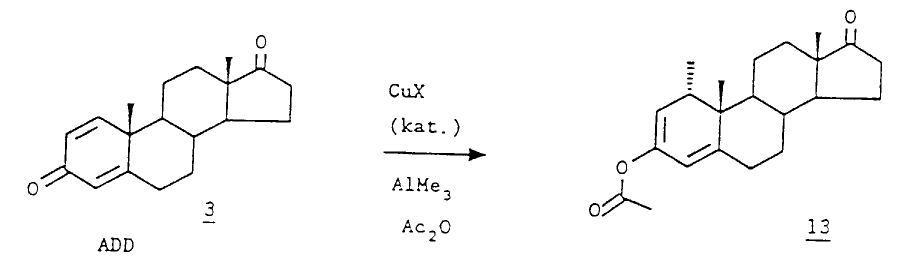

3-Acetoxy-1α-methyl-androsta-2,4-dien-17-on (13) ist ein wichtiges Zwischenprodukt für die Synthese von Atamestan, welches daraus durch stereoselektive 2β-Iodierung sowie nachfolgende Iodwasserstoffabspaltung in hoher Ausbeute erhalten wird (Deutsche Patentanmeldung P 40 15 247.2 und DE-A-37 15 869.4).

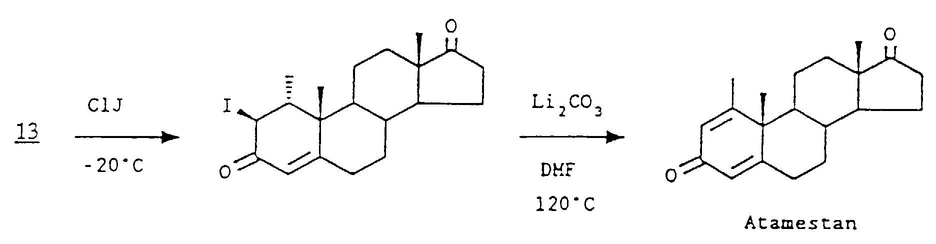

Das Verfahren ist auch geeignet, eine 1,4-Addition an ein einfach ungesättigtes Carbonylsystem wie z.B. 14 einzugehen.

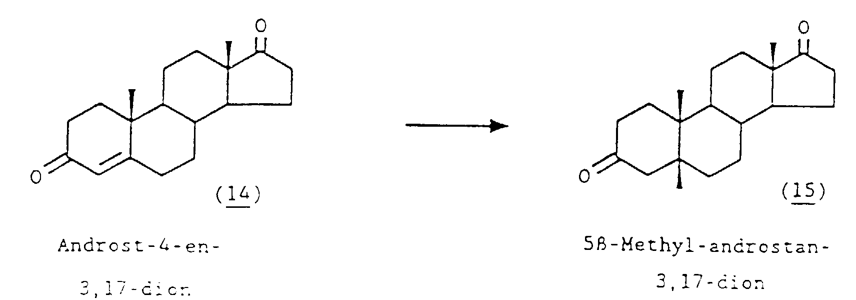

Aus Androst-4-en-3,17-dion (14) entsteht hierbei 5β-Methyl-androstan-3,17-dion (15).

Über die oben aufgezeigte 1,4-Addition an α,β-ungesättigte Keto-Steroide hinaus ist das erwähnte Verfahren ganz allgemein zur 1,4-Addition einer Methyl- bzw. Ethylgruppe an ein α,β-ungesättigtes Keton anwendbar. Ein Beispiel dazu ist die Umsetzung von Cyclohex-2-en-1-on (16) zu 3-Methylhexanon (17).

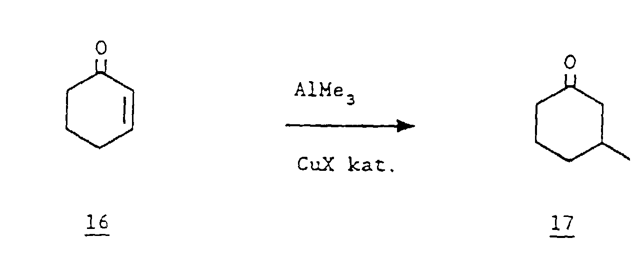

Alle Reaktionen werden vorzugsweise zwischen 0 °C und 50 °C durchgeführt. Zur Reaktion wird das Keton bzw. Ketosteroid in einem geeigneten Lösungsmittel unter Zusatz von 5 - 10 Mol-% Kupfer-Katalysator unter einer Atmosphäre von Inertgas wie z.B. Stickstoff vorgelegt und Aluminiumtrimethyl (Zinkdimethyl, Aluminiumtriethyl) zwischen 0 °C und Raumtemperatur zugegeben. Die Reaktion wird nach ca. 30 - 120 Minuten unter Zusatz von Wasser oder einem niederen Alkohol hydrolysiert und das Produkt anschließend isoliert.

Das erwähnte Mittel und das beschriebene Verfahren sind nicht auf den Einsatz von Trimethylaluminium oder Dimethylzink bzw. Triethylaluminium als Methyl- bzw. Ethylquelle beschränkt. Ganz analog wie vorstehend beschrieben läßt sich anstelle der genannten Alkylierungs-Reagenzien auch ein Aluminium-Reagenz der Formel Alk₃₋ₘ AlOEtₘ, worin Alk eine Methyl- oder Ethylgruppe und OEt eine Ethoxygruppe bedeuten und m gleich 1 oder 2 ist, als das die Methyl- bzw. Ethylgruppe liefernde Reagenz innerhalb des erfindungsgemäßen Mittels oder Verfahrens verwenden. Wenn Alk für eine Methylgruppe steht, so ist m vorzugsweise 1 (Dimethylaluminium-ethoxid).

Das erwähnte Mittel sowie das entsprechende Verfahren lassen sich sehr breit anwenden, was beispielsmäßig durch die Herstellung folgender Verbindungen aus den entsprechenden Enonen demonstriert wird:
1α-Methylandrost-4-en-3,17-dion
5β-Methyl-19-norandrostan-3,17-dion
1α-Methyl-17β-acetoxy-androst-4-en-3-on
5β-Methylandrostan-3,17-dion
17β-Hydroxy-1α-methyl-androst-4-en-3-on (1α-Methyltestosteron)
17β-Acetoxy-1α-methyl-5α-androstan-3-on
16α-Methyl-pregna-1,4-dien-20-on
3-Acetoxy-1α-methyl-androsta-2,4-dien-17-on
3-Methylcyclohexanon
1α:-Methyl-androsta-4,6-dien-3,17-dion
3-Acetoxy-16a-methyl-pregn-5-en-20-on
la-Ethylandrost-4-en-3,17-dion
la-Ethylandrosta-4,6-dien-3,17-dion
3-Acetoxy-16α-ethyl-pregn-5-en-20-on
4-Phenyl-pentan-2-on
4-Phenyl-hexan-2-on
1α-Methylandrost-4-en-3,17-dion
2β-Iod-1α-methylandrost-4-en-3,17-dion(→ Atamestan)
17β-Acetoxy-2α-brom-1α-methyl-5α-androstan-3-on
17-Acetoxy-1α-ethyl-5α-androstan-3-on
1-Methyl-7,7-(2,2-dimethyltrimethylendioxy)-cis-bicyclo[3.3.0]octan-3-on
1-Ethyl-7,7-(2,2-dimethyltrimethylendioxy)-cis-bicyclo[3.3.0]octan-3-on
2-tert.-Butyl-5-methyl-cyclohexanon
3,3,5,5-Tetramethylcyclohexanon
17β-Acetoxy-1α-methyl-5α-androstan-3-on

Es wurde nunmehr gefunden, daß sich das vorstehend beschriebene Alkylierungsmittel und das Verfahren unter dessen Verwendung sogar noch verbessern lassen, wenn diesem Alkylierungsmittel ein (oder mehrere) Silylreagenz(ien) der allgemeinen Formel III

R¹R²R³SiZ (III),

worin
R¹, R² und R³ gleich oder unterschiedlich sein können und einen gerad- oder verzweigtkettigen Alkylrest mit 1, bzw. im verzweigtkettigen Fall mit 3, bis 10 Kohlenstoffatomen, einen gegebenenfalls mit 1 bis 3 Chloratomen oder 1 bis 3 gerad- oder verzweigtkettigen Alkoxy- bzw. Alkylresten mit 1 bzw. 3 bis 6 Kohlenstoffatomen substituierten Arylrest sowie
Z ein Chlor-, Brom- oder Iodatom, den Cyano-, einen Perfluoralkylsulfonyloxyrest [(CₙF₂ₙ₊₁SO₂O-), mit n = 1,2,3 oder 4], den Mesylatrest CH₃SO₂O- oder den Tolsylrest p-CH₃-C₆H₄-SO₂O- bedeuten, zugesetzt wird.

Außerdem wurde noch gefunden, daß in dem neuen Alkylierungsmittel gemäß vorliegender Erfindung nicht nur Trimethylaluminium oder Dimethylzink bzw. Triethylaluminium oder eine Verbindung der Formel Alk₃₋ₘ AlOEtₘ, worin Alk eine Methyl- oder Ethylgruppe bedeutet, als Alkylquelle dienen können, Sonden daß generell ein Aluminiumreagenz der Formel Alk₃₋ₘ AILₘ, worin Alk eine Methyl-, Ethyl-, n- oder i-Propyl-, n-, i-oder tert.-Butyl-, Pentyl-, Hexyl-, Heptyl- oder Octylgruppe, die alle auch verzweigt sein können, L eine Ethoxygruppe, ein Chlor- oder Bromatom bedeuten und m gleich 0, 1 oder 2 ist, als Alkylquelle in einem derartigen Mittel geeignet ist und daß mit einem derartigen Mittel auch die Einführung dieser höheren homologen Alkylreste in ein α,β-ungesättigtes oder ein α,β-doppelt ungesättigtes Keton oder in einen α,β-ungesättigten Aldehyd gelingt.

Die vorliegende Erfindung betrifft daher ein derartig verbessertes, silylhaltiges Alkylierungsmittel sowie ein Verfahren zur 1,4-Addition einer Alkylgruppe Alk (Alk ist eine Methyl-, Ethyl-, n- oder i-Propyl-, n-, i-oder tert.-Butyl-, Pentyl-, Hexyl-, Heptyl- oder Octylgruppe) unter verwendung des verbesserten silylhaltigen Alkylierungsmittels.

Als Alkylquelle sind erfindungsgemäß Dimethylzink, Trimethylaluminium, Dimethylaluminiumchlorid, Dimethylaluminiumethoxid oder Triethylaluminium bevorzugt.

Als gerad- oder verzweigtkertige Alkylreste R¹, R², R³ kommen beispielsweise der Methyl-, Ethyl-, Propyl-, Butyl- und der tert.-Butylrest infrage, vorzugsweise der Methyl- und der tert.-Butylrest.

Ein unsubstituierter Phenylrest ist als Arylrest bevorzugt, jedoch ist ohne weiteres auch ein o-, m- oder p-Tolyl- oder ein Xylylrest als substituierter Arylrest denkbar.

Von den für Z genannten Substituenten ist ein Chloratom oder auch eine Triflat-Gruppe (CF₃SO₂O-) bevorzugt.

Die erfindungsgemäß bevorzugten Silylreagenzien sind die gängigen Silylreagenzien
Trimethylsilylchlorid (TMSCI)
Tert.-Butyl-dimethylsilylchlorid (TBDMSCI)
Tert.-Butyl-diphenylsilylchlorid (TBDPSCI)
Trimethylsilyltriflat und
Trimethylsilylcyanid (TMSCN).
Insbesondere bevorzugt ist Trimethylsilylchlorid.

Das neue Alkylierungsmittel enthält das Silylreagenz der allgemeinen Formel III im allgemeinen in einer Konzentration von 10 - 1000 Mol-%, vorzugsweise 50 - 300 Mol-% und insbesondere 100 - 250 Mol-% bezogen auf die zu alkylierende Verbindung.

Ansonsten unterscheidet sich das neue Alkylierungsmittel nicht von dem erwähnten, vorstehend beschriebenen Alkylierungsmittel der nicht-vorveröffentlichten europäischen Patentanmeldung Nr. 92250276.0 (Veröffentlichungs-Nr. 0534582) und das neue Verfahren wird ganz analog wie vorstehend und in der genannten europäischen Patentanmeldung beschrieben, nunmehr aber unter Verwendung des neuen, zusätzlich ein Silylreagenz der allgemeinen Formel III enthaltenden Alkylierungsmittels durchgeführt.

Es wurde aber gefunden, daß sowohl das nicht-vorveröffentlichte als auch das neue Alkylierungsmittel noch weniger Kupfer-I- und/oder Kupfer-II-verbindung enthalten können, nämlich insgesamt 0,1 - 10 Mol-% bezogen auf die zu alkylierende Verbindung. Vorzugsweise sind im neuen Alkylierungsmittel ingesamt 1 - 10 Mol-% Cu-I- und/oder Cu-II-Verbindung bezogen auf die zu alkylierende Verbindung enthalten.

Durch den Zusatz des Silylreagenzes der allgemeinen Formel III, insbesondere wird Trimethylsilylchlorid verwendet, wird vor allem die 1,4-Addition einer Methyl- oder Ethylgruppe an 3-Keto-Δ^{1,4}-Steroide beschleunigt, jedoch ist das neue Alkylierungsmittel und dessen Verwendung nicht auf Steroide beschränkt.

Neben einer Beschleunigung der Reaktion wird durch den Zusatz einer (oder mehrerer verschiedener) Silylreagenzien der allgemeinen Formel III auch die Bildung von Folgeprodukten verringert sowie der Anteil an Ausgangsmaterial im Rohprodukt auf unter 1 % reduziert.

Die Ausbeute an Alkylierungsprodukt kann dadurch wesentlich gesteigert werden, beispielsweise bei der Darstellung von
1α-Methylandrost-4-en-3,17-dion von 77 % auf 89 % der Theorie (vgl. Beispiel 1 und Beispiel 8 als Vergleichsbeispiel),
1α-Ethylandrost-4-en-3,17-dion von 85 % auf 90 % der Theorie (vgl. Beispiel 2 und Beispiel 9 als Vergleichsbeispiel) und
2-tert.-Butyl-5-methylcyclohexanon von 70 % auf 86 % der Theorie (vgl. Beispiel 4 und Beispiel 10 als Vergleichsbeispiel).

Der Zusatz von Silylchloriden ist bei anderen Organometallverbindungen (Cupraten usw.) bereits bekannt und unter folgenden Literaturstellen beschrieben worden:
a) C.R. Johnson, T.J. Marren, Tetrahedron Lett., 1987, 28, 27.
b) E. Nakamura S. Matsuzawa, Y. Horiguchi, I. Kuwajima, Tetrahedron Lett., 1986, 27 4029.
c) C. Chuit, J.P. Foulon, J.F. Normant, Tetrahedron 1980, 36, 2305 ; Tetrahedron 1981, 37, 1385.
d) E.J. Corey, N.W. Boaz, Tetrahedron Lett., 1985, 6015; ibid 1619.
e) A. Alexakis, J. Berlan, Y. Besace, Tetrahedron Lett., 1986, 27, 1047.

Einen Übersichtsartikel betreffend den Zusatz von Lewis-Säuren wie Trimethylsilylchlorid usw. bei 1,4-Additionen findet man von Y. Yamamoto in Angew. Chem. 1986, 98, 945.

Es wurde auch BF₃Et₂O als Lewis-Säure-Zusatz zum erwähnten Alkylierungsmittel (europäische Patentanmeldung Nr. 92250276.0) untersucht. Dieser Zusatz führt zu keiner Verbesserung des Reaktionsverlaufes.

Bei den Reaktionen mit Trimethylsilyl-Zusatz entstehen intermediär Silylenolether, die bei der Aufarbeitung hydrolysiert werden. Bei Aufarbeitung unter milden Bedingungen oder bei Verwendung von Silylchloriden, die hydrolysestabilere Silylenolether ergeben, lassen sich diese Silylenolether als Reaktionsprodukte auch isolieren.

Die nachstehenden Beispiele dienen der näheren Erläuterung der Erfindung. Beispiele 8, 9 und 10 sind Vergleichsbeispiele aus der europäischen Patentanmeldung Nr. 92250276.0, die ohne den Zusatz eines Silylreagenzes ebenfalls, aber in schlechteren Ausbeuten, zu den Produkten der erfindungsgemäßen Beispiele 1.2 und 4 führen.

### Beispiel 1: 1α-Methylandrost-4-en-3,17-dion

Unter Stickstoff werden 8.52 g (30 mmol) Androsta-1,4-dien-3,17-dion (1) und 86 mg (0.6 mmol) CuBr in 70 ml THF gelöst. Unter Eisbadkühlung werden 28,4 ml (33 mmol) einer Lösung Trimethylaluminium 10%ig in Toluol zugegeben. Zu der Lösung werden 3.26 g (30 mmol) Trimethylsilylchlorid zugegeben. Die Lösung noch 2 h bei RT gerührt. Die Lösung wird mit 3 ml Wasser hydrolysiert, der anorganische Feststoff abgesaugt und nachgewaschen. Chromatographie des Rohproduktes an Silicagel mit Ethylacetat/ Hexan ergibt 8 g Produkt 1α-Methylandrost-4-en-3,17-dion (89% der Theorie) vom Schmelzpunkt 154°C.

### Beispiel 2: 1α-Ethylandrost-4-en-3,17-dion

Unter Stickstoff werden 2.84 g (10 mmol) Androsta-1,4-dien-3,17-dion (1) und 143 mg (1 mmol) CuBr in 15 ml THF gelöst. Bei 20°C werden 5.78 ml (11 mmol) einer Lösung Triethylaluminium 1.9 molar in Toluol zugegeben. Zu der Lösung werden 2.16 g (20 mmol) Trimethylsilylchlorid gegeben. Die Lösung wird noch 3.5 h bei RT gerührt. Die Lösung wird mit 3 ml Wasser hydrolysiert, der anorganische Feststoff abgesaugt und mit Ethylacetat nachgewaschen. Chromatographie des Rohproduktes an Silicagel mit Ethylacetat/Hexan als Eluens ergibt 2.8 g 1α-Ethylandrost-4-en-3,17-dion (89% der Theorie) vom Schmelzpunkt 168°C.

### Beispiel 3: 17β-Acetoxy-la-ethylandrostan-3-on

3.31 g (10 mmol) 17β-Acetoxy-androst-1-en-3-on und 143 mg (1 mmol) CuBr werden in 15 ml trockenem THF vorgelegt. Bei 0° C werden 5.79 ml (11 mmol) einer 1.9 molaren Lösung Triethylaluminium in Toluol sowie 2.16 g (20 mmol) Trimethylsilylchlorid zugegeben. Es wird 1 h bei Raumtemperatur gerührt, anschließend mit 10 ml 2n Salzsäure hydrolysiert und das Produkt mit Methyltertiärbutyl-ether extrahiert. Nach Abdampfen des Lösungsmittels und Umkristallisation aus Aceton werden 3.2 g 17β-Acetoxy-1α-ethylandrostan-3-on (90% der Theorie) vom Schmelzpunkt 159° C erhalten.

### Beispiel 4: 2-tert.-Butyl-5-methylcyclohexanon

Bei Raumtemperatur werden 1.52 g (10 mmol) Pulegon (2-Isopropyliden-5-methylcyclohexanon) und 14,3 mg (0.1 mmol) CuBr in 10 ml trockenem THF vorgelegt. Bei 0°C werden 9.5 ml (11 mmol) Trimethylaluminium 10%ig in Toluol sowie 1.3 g (12 mmol) Trimethylsilylchlorid zugegeben. Es wird noch 5 h bei Raumtemperatur gerührt, mit 5 ml Wasser hydrolysiert und 2 x mit je 30 ml Ether extrahiert und die organischen Phasen vereint. Nach Abdampfen des Lösungsmittels wird das Produkt bei 10 Torr und 120 °C im Kugelrohr destilliert. Es werden 1.45 g (86% der Theorie) 2-tert.-Butyl-5-methylcycolohexanon erhalten.

### Beispiel 5: 3,3-Dimethylcyclohexanon

1.1 g (10 mmol) 3-Methyl-cyclohex-2-en-1-on und 28 mg (0.2 mmol) CuBr werden in 15 ml THF vorgelegt. Bei 0° C werden 11 ml (11 mmol) einer Lösung Trimethylaluminium 10%ig in Hexan sowie 2.16 g (20 mmol) Trimethylsilylchlorid zugegeben und 3 h bei Raumtemperatur gerührt. Es wird mit 10 ml 1 n Salzsäure hydrolysiert, mit Ethylacetat extrahiert und das Lösungsmittel abgedampft. Es werden 1.21 g (97% der Theorie) 3,3-Dimethylcyclohexanon erhalten.

### Beispiel 6: 3-Ethyl-3-methylcyclohexanon

1.1 g (10 mmol) 3-Methyl-cyclohex-2-en-1-on und 14 mg (0.2 mmol) CuBr werden in 15 ml THF vorgelegt. Bei 0°C werden 5.78 ml (11 mmol) einer 1.6 molaren Lösung Triethylaluminium in Toluol sowie 2.16 g (20 mmol) Trimethylsilylchlorid zugegeben und 3 h bei Raumtemperatur gerührt. Es wird mit 10 ml 1 n Salzsäure hydrolysiert, mit Ethylacetat extrahiert und das Lösungsmittel abgedampft. Nach Chromatographie an Kieselgel werden 1.26 g (90 % der Theorie) 3-Ethyl-3-methyl-cyclohexanon erhalten.

### Beispiel 7: 1α-Methylandrost-4-en-3,17-dion

Unter Stickstoff werden 5,68 g (20 mmol) Androsta-1,4-dien-3,17-dion (1) und 143 mg ( 1 mmol) CuBr in 50 ml THF gelöst. Unter Eisbadkühlung werden 22 ml (22 mmol) einer Lösung Dimethylaluminiumchlorid als 10%ige Hexan-Lösung zugegeben. Zu der Reaktionslösung werden 2,6 g g (24 mmol) Trimethylsilylchlorid zugegeben. Die Lösung wird noch 2 h bei RT gerührt. Zur Hydrolyse wird die Lösung mit 15 ml 1 molare HCl-Lösung versetzt und 15 Minuten nachgerührt. Das Produkt wird 3 x mit je 40 ml Ethylacetat extrahiert. Chromatographie des Rohproduktes an Silicagel mit Ethylacetat/ Hexan als Eluens ergibt als Produkt 4.9 g 1α-Methylandrost-4-en-3,17-dion (82% der Theorie) vom Schmelzpunkt 152-154°C.

### Beispiel 8: 1α-Methylandrost-4-en-3,17-dion

14.2 g (50 mmol) Androsta-1,4-dien-3,17-dion werden unter Stickstoffatmosphäre in 100 ml wasserfreiem Dioxan gelöst. 716 mg (5 mmol) Kupfer-I-bromid werden zugegeben und die Lösung auf 25 °C erwärmt. Anschließend werden 47 ml (55 mmol) einer 10% igen Lösung Trimethylaluminium in Toluol zu der Reaktion gegeben, so daß die Temperatur nicht über 35 °C ansteigt. Anschließend wird noch 1.5 h bei 35°C nachgerührt. Zur Hydrolyse werden 2.5 ml Wasser gemischt mit 10 ml Dioxan zur Reaktion gegeben und die Lösung noch 15 min nachgerührt. Der anorganische Feststoff wird abgesaugt und mit 30 ml Dioxan nachgewaschen. Nach Einengen der Dioxanlösung werden 17 g Rohprodukt erhalten, die an Kieselgel mit Hexan/Ethylacetat-Gemischen als Eluens chromatographiert werden. Nach Einengen der Fraktionen und Umkristallisation aus Diisopropylether werden 11.66 g 1α-Methylandrost-4-en-3,17-dion (77 % der Theorie) vom Schmelzpunkt 154 °C erhalten.

### Beispiel 9: 1α-Ethylandrost-4-en-3,17-dion

2.84 g (10 mmol) Androsta-1,4-dien-3,17-dion werden unter Stickstoffatmosphäre in 20 ml wasserfreiem Dioxan gelöst. 143 mg (1 mmol) Kupfer-I-bromid werden zugegeben und die Lösung auf 25 °C erwärmt. Anschließend werden 10 ml (10 mmol) einer 1 molaren Lösung Triethylaluminium in Hexan zu der Reaktion gegeben, so daß die Temperatur nicht über 30 °C ansteigt. Anschließend wird noch 1.5 h bei 30 °C nachgerührt. Zur Hydrolyse wird 1 ml Wasser gemischt mit 5 ml Dioxan zur Reaktion gegeben und die Lösung noch 15 min gerührt. Der anorganische Feststoff wird abgesaugt und mit 30 ml Dioxan nachgewaschen. Nach Einengen der Dioxanlösung werden 3 g Rohprodukt erhalten, die an Kieselgel mit einem Hexan/Ethylacetat- Gemisch unter steigendem Anteil Ethylacetat als Eluens chromatographiert werden. Nach Einengen der Fraktionen werden 2.67 g 1α-Ethylandrost-4-en-3,17-dion (85 % der Theorie vom Schmelzpunkt 168°C erhalten.

### Beispiel 10: 2-tert.-Butyl-5-methyl-cyclohexanon

Zu 4.56 g (30 mmol) 2-Isopropyliden-5-methyl-cyclohexan-1-on (Pulegon) und 214.5 mg (1.5 mmol) CuBr in 30 ml Ethylacetat werden 28.5 ml (33 mmol) Trimethylaluminium als 10%ige Lösung in Toluol getropft. Die Reaktionslösung wird noch 1h bei 25 °C gerührt. Zur Hydrolyse werden vorsichtig 2 ml Wasser zugegeben und noch 15 min nachgerührt. Der anorganische Feststoff wird abgesaugt, mit Ethylacetat nachgewaschen und die Lösung im Vakuum eingeengt. Destillation des Rohproduktes bei 120°C/6 Torr ergibt 3.4 g 2-tert-Butyl-5-methyl-cyclohexanon (70% der Theorie) als Isomerengemisch.

## Patentansprüche

1. Alkylierungsmittel, enthaltend ein Aluminium-Reagenz Alk₃₋ₘAILₘ, worin Alk eine Methyl-, Ethyl-, n- oder i-Propyl-, n-, i- oder ter.-Butyl-, Pentyl-, Hexyl-, Heptyl- oder Octylgruppe, die alle auch verzweigt sein können, L eine Ethoxygruppe, ein Chlor- oder Bromatom bedeuten und m bleich 0, 1 oder 2 ist, als Alkylquelle oder auch Zinkdimethyl als Methylquelle, dadurch gekennzeichnet, daß es zusätzlich katalytische Mengen einer oder mehrerer Kupfer-I- und/oder Kupfer-II-Verbindungen und ein (oder mehrere) Silylreagenz(ien) der allgemeinen Formel III
R¹R²R³SiZ (III),
worin
R¹, R² und R³ gleich oder unterschiedlich sein können und einen gerad- oder verzweigtkettigen Alkylrest mit 1, bzw. im verzweigtkettigen Fall mit 3, bis 10 Kohlenstoffatomen, einen gegebenefalls mit 1 bis 3 Chloratomen oder 1 bis 3 gerad- oder verzweigtkettigen Alkoxy- bzw. Alkylresten mit 1, bzw. im verzweigtkettigen Fall 3, bis 6 Kohlenstoffatomen substituierten Arylrest sowie
Z ein Chlor-, Brom- oder Iodatom, den Cyano-, einen Perfluoralkylsulfonyloxyrest [(CₙF₂ₙ₊₁SO₂O-), mit n = 1, 2, 3 oder 4], den Mesylatrest CH₃SO₂O- oder den Tosylrest p-CH₃-C₆H₄-SO₂-O- bedeuten, enthält.

2. Methylierungsmittel nach Anspruch 1, dadurch gekennzeichnet, daß es Dimethylzink, Trimethylaluminium, Dimethylaluminiumchlorid oder Dimethylaluminiumethoxid als Methylquelle enthält.

3. Ethylierungsmittel nach Anspruch 1, dadurch gekennzeichnet, daß es Triethylaluminium als Ethylquelle enthält.

4. Alkylierungsmittel nach Anspruch 1, 2 oder 3, dadurch gekennzeichnet, daß es als Kupfer-I und/oder Kupfer-II-Verbindung eine oder mehrere Verbindung(en) der allgemeinen Formel I
CuX oder CuX₂ (I),
worin X einen einwertigen Rest darstellt und für Chlor, Brom, Iod, Cyano, der Thienyl-, Phenyl-, einen Alkoxy-, Thioalkoxy-, wobei der darin enthaltene Alkylrest 1 bis 8 Kohlenstoffatome besitzt und gegebenenfalls verzweigt und/oder ungesättigt ist, für einen substituierten Alkinylrest R-C≡C-, wobei R einen Phenyl- oder einen gegebenenfalls verzweigten C₁-C₈-Alkylrest bedeutet, oder den Rest einer anorganischen Säure oder einer Carbonsäure oder für einen zweizähnigen, über Sauerstoff- und/oder Stickstoffatome koordinierenden Komplexliganden, ausgenommen den Liganden Acetylacetonat im Fall des zweiwertigen Kupfers steht und/oder eine oder mehrere Verbindung(en) der allgemeinen Formel II
Cu₂Y oder CuY (II),
worin Y einen zweiwertigen Rest darstellt und für Sauerstoff oder Schwefel steht, enthält.

5. Alkylierungsmittel nach Anspruch 4, dadurch gekennzeichnet, daß es Kupfer-I- und/oder Kupfer-II-chlorid und/oder -bromid und/oder Kupfer-I-cyanid enthält.

6. Alkylierungsmittel nach einem der vorstehenden Ansprüche, dadurch gekennzeichnet, daß es insgesamt 0,1 bis 10 Mol-% der Kupfer-I- und/oder Kupfer-II-verbindung bezogen auf die zu alkylierende Verbindung enthält.

7. Alkylierungsmittel nach einem der vorstehenden Ansprüche, dadurch gekennzeichnet, daß es Trimethylsilylchlorid, tert.-Butyl-dimethylsilylchlorid. tert.-Butyldiphenylsilylchlorid, Trimethylsilyltriflat und/oder Trimethylsilylcyanid als Silylreagenz(ien) enthält.

8. Alkylierungsmittel nach einem der vorstehenden Ansprüche, dadurch gekennzeichnet, daß es insgesamt 10 bis 1000 Mol-% des Silylreagenzes bezogen auf die zu alkvlierende Verbindung enthält.

9. Verfahren zur 1,4-Addition einer Alkylgruppe Alk (Alk ist eine Methyl-, Ethyl-, n- oder i-Propyl-, n-, i- oder tert.-Butyl-, Pentyl-, Hexyl-, Heptyl- oder Octyl- gruppe, die alle auch verzweigt sein können) an ein α,β-ungsättigtes oder ein α,β-doppelt ungesättigtes Keton oder einen α,β-ungesättigten Aldehyd, dadurch gekennzeichnet, daß das α,β-ungesättigte oder das α,β-doppelt ungesättigte Keton oder der α,β-ungesättigte Aldehyd mit einem Aluminium-Reagenz Alk₃₋ₘAILₘ, worin Alk eine Methyl-, Ethyl-, n- oder i-Propyl-, n-, i- oder tert.-Butyl-, Pentyl-, Hexyl-, Heptyl- oder Octylgruppe, die alle auch verzweigt sein können, L eine Ethoxygruppe, ein Chlor- oder Bromatom bedeuten und m gleich 0, 1 oder 2 ist, in Gegenwart einer katalytischen Menge einer oder mehrerer Kupfer-I- und/oder Kupfer-II-Verbindungen und eines (oder mehrerer) Silylreagenzes (-reagenzien) der allgemeinen Formel III
R¹R²R³SiZ (III),
worin
R¹, R² und R³ gleich oder unterschiedlich sein können und einen gerad- oder verzweigtkettigen Alkylrest mit 1, bzw. im verzweigtkettigen Fall mit 3, bis 10 Kolhlenstoffatomen, einen gegebenenfalls mit 1 bis 3 Chloratomen oder 1 bis 3 gerad- oder verzweigtkettigen Alkoxy- bzw. Alkylresten mit 1, bzw. im verzweigtkettigen Fall 3, bis 6 Kohlenstoffatomen substituierten Arylrest sowie
Z ein Chlor-, Brom- oder Iodatom, den Cyano-, einen Perfluoralkylsulfonyloxyrest [(CₙF₂ₙ₊₁SO₂O-), mit n = 1, 2, 3 oder 4], den Mesylatrest CH₃SO₂O- oder den Tosylrest p-CH₃-C₆H₄-SO₂O- bedeuten, alkyliert wird.

10. Verfahren nach Anspruch 9, dadurch gekennzeichnet, daß mit Dimethylzink, Trimethylaluminium, Dimethylaluminiumchlorid oder Dimethylaluminiumethoxid methyliert wird.

11. Verfahren nach Anspruch 9, dadurch gekennzeichnet, daß mit Triethylaluminium ethyliert wird.

12. Verfahren nach Anspruch 9, 10 oder 11, dadurch gekennzeichnet, daß in Gegenwart einer oder mehrerer verbindung(en) der allgemeinen Formel I
CuX oder CuX₂ (I),
worin X einen einwertigen Rest darstellt und für Chlor, Brom, Iod, Cyano, den Thienyl-, Phenyl-, einen Alkoxy-, Thioalkoxy-, wobei der darin enthaltene Alkylrest 1 bis 8 Kohlenstoffatome besitzt und gegebenenfalls verzweigt und/oder ungesättigt ist, für einen substituierten Alkinylrest R-C≡C-, wobei R einen Phenyl- oder einen gegebenenfalls verzweigten C₁-C₈-Alkylrest bedeutet, oder den Rest einer anorganischen Säure oder einer Carbonsäure oder für einen zweizähnigen, über Sauerstoff- und/oder Stickstoffatome koordinierenden Komplexliganden. ausgenommen den Liganden Acetylacetonat im Fall des zweiwertigen Kupfers steht und/oder eine oder mehrere Verbindung(en) der allgemeinen Formel II
Cu₂Y oder CuY (II),
worin Y einen zweiwertigen Rest darstellt und für Sauerstoff oder Schwefel steht, alkyliert wird.

13. Verfahren nach Anspruch 12, dadurch gekennzeichnet, daß in Gegenwart von Kupfer-I- und/oder Kupfer-II-chlorid und/oder -bromid und/oder Kupfer-I-cyanid alkyliert wird.

14. Verfahren nach einem der vorstehenden Ansprüche 9 bis 13, dadurch gekennzeichnet, daß in Gegenwart von insgesamt 0,1 - 10 Mol-% der Kupfer-I- und/oder Kupfer-II-Verbindung bezogen auf die zu alkylierende α,β-ungesättigte Verbindung alkyliert wird.

15. Verfahren nach einem der Ansprüche 9 bis 14, dadurch gekennzeichnet, daß in Gegenwart von Trimethylsilylchlorid, tert.-Butyl-dimethylsilylchlorid, tert.-Butyldiphenylsilylchlorid, Trimethylsilyltriflat und/oder Trimethylsilylcyanid als Silylreagenz(ien) alkyliert wird.

16. Verfahren nach einem der vorstehenden Ansprüche 9 bis 15, dadurch gekennzeichnet, daß in Gegenwart von insgesamt 10 - 1000 Mol-% des Silylreagenzes bezogen auf die zu alkylierende Verbindung alkyliert wird.

17. Verfahren nach einem der vorstehenden Ansprüche 9 bis 16, dadurch gekennzeichnet, daß es in Tetrahydrofuran, Dioxan, Dimethoxyethan oder Toluol als Lösungsmittel durchgeführt wird.

18. Verfahren nach einem der vorstehenden Ansprüche 9 bis 16, dadurch gekennzeichnet, daß es in Ethylacetat als Lösungsmittel durchgeführt wird.

19. Verfahren nach einem der vorstehenden Ansprüche 9 bis 18, dadurch gekennzeichnet, daß es bei einer Reaktionstemperatur von 0 °C bis 50 °C durchgeführt wird.

20. Verfahren nach einem der vorstehenden Ansprüche 9 bis 19, dadurch gekennzeichnet, daß das α,β-ungesättigte Keton ein 3-Keto-1,4-dien-Steroid, ein 3-Keto-1-en-Steroid, ein 3-Keto-4-en-Steroid oder ein 17-Acyl-16-en-Steroid ist.

21. Verfahren zur Herstellung von 1α-Methylandrost-4-en-3,17-dion als Zwischenprodukt zur Herstellung von 1α-Methyl-17β-hydroxy-5α-androstan-3-on (Mesterolon), dadurch gekennzeichnet, daß Androsta-1,4-dien-3,17-dion gemäß einem der Ansprüche 9 bis 19 methyliert wird.

22. Verfahren zur Herstellung eines 3-Acyloxy-1α-methyl-androsta-2,4-dien-17-ons als Zwischenprodukt für die Herstellung von 1-Methyl-androsta-1,4-dien-3,17-dion (Atamestan), dadurch gekennzeichnet, daß Androsta-1,4-dien-3,17-dion gemäß einem der Ansprüche 9 bis 19 methyliert und das nach der Methylierung in der Reaktionsmischung vorliegende Enolat mit einem Carbonsäureanhydrid- oder -chlorid einer gerad- oder verzweigtkettigen Alkancarbonsäure mit 2 bis 8 Kohlenstoffatomen oder der Benzoesäure, abgefangen wird.

23. Verfahren nach Anspruch 22, dadurch gekennzeichnet, daß das Enolat mit Acetanhydrid oder Acetylchlorid abgefangen wird.

## Claims

1. Alkylating agent, comprising as source of alkyl an aluminium reagent Alk₃₋ₘAlLₘ, wherein Alk is a methyl, ethyl, n-propyl or isopropyl, n-butyl, isobutyl or tert-butyl, pentyl, hexyl, heptyl or octyl group, all of which may also be branched, L is an ethoxy group or a chlorine or bromine atom and m is 0, 1 or 2, or comprising zinc dimethyl as source of methyl, characterised in that it comprises additionally catalytic amounts of one or more copper(I) and/or copper(II) compound(s) and one (or more) silyl reagent(s) of the general formula III
R¹R²R³SiZ (III),
wherein
each of R¹, R² and R³ may be the same or different and is a straight-chained or branched-chained alkyl radical having from 1, or in the case of a branched chain from 3, to 10 carbon atoms, or an aryl radical that is optionally substituted by from 1 to 3 chlorine atoms or by from 1 to 3 straight-chained or branched-chained alkoxy or alkyl radicals having from 1, or in the case of a branched chain from 3, to 6 carbon atoms, and
Z is a chlorine, bromine or iodine atom, cyano, a perfluoroalkylsulphonyloxy radical [(CₙF₂ₙ₊₁SO₂O-) in which n is 1, 2, 3 or 4], the mesylate radical CH₃SO₂O- or the tosyl radical p-CH₃-C₆H₄-SO₂O-.

2. Methylating agent according to claim 1, characterised in that it comprises dimethylzinc, trimethylaluminium, dimethylaluminium chloride or dimethylaluminium ethoxide as source of methyl.

3. Ethylating agent according to claim 1, characterised in that it comprises triethylaluminium as source of ethyl.

4. Alkylating agent according to claim 1, 2 or 3, characterised in that it comprises as copper(I) and/or copper(II) compound one or more compound(s) of the general formula I
CuX or CuX₂ (I)
wherein X represents a monovalent radical and is chlorine, bromine, iodine, cyano, a thienyl radical, a phenyl radical, an alkoxy or thioalkoxy radical wherein the alkyl radical contained therein has from 1 to 8 carbon atoms and is optionally branched and/or unsaturated, a substituted alkynyl radical R-C≡C- wherein R is a phenyl radical or an optionally branched C₁-C₈alkyl radical, or the radical of an inorganic or carboxylic acid, or, with the exception of the acetylacetonate ligand in the case of the bivalent copper, X is a bidentate complex ligand that co-ordinates by way of oxygen and/or nitrogen atoms,
and/or one or more compound(s) of the general formula II
Cu₂Y or CuY (II)
wherein Y represents a bivalent radical and is oxygen or sulphur.

5. Alkylating agent according to claim 4, characterised in that it comprises copper(I) and/or copper(II) chloride and/or bromide and/or copper(I) cyanide.

6. Alkylating agent according to any one of the preceding claims, characterised in that it comprises a total of from 0.1 to 10 mol % of the copper(I) and/or copper(II) compound, based on the compound that is to be alkylated.

7. Alkylating agent according to any one of the preceding claims, characterised in that it comprises as silyl reagent(s) trimethylsilyl chloride, tert-butyldimethylsilyl chloride, tert-butyldiphenylsilyl chloride, trimethylsilyltriflate and/or trimethylsilyl cyanide.

8. Alkylatine agent according to any one of the preceding claims, characterised in that it comprises a total of from 10 to 1000 mol % of the silyl reagent, based on the compound that is to be alkylated.

9. Process for the 1,4-addition of an alkyl group Alk (Alk is a methyl, ethyl, n-propyl or isopropyl, n-butyl, isobutyl or tert-butyl, pentyl, hexyl, heptyl or octyl group, all of which may also be branched) to an α,β-unsaturated or α,β-doubly-unsaturated ketone or to an α,β-unsaturated aldehyde, characterised in that the α,β-unsaturated ketone, the α,β-doubly-unsaturated ketone or the α,β-unsaturated aldehyde is alkylated with an aluminium reagent Alk₃₋ₘAlLₘ, wherein Alk is a methyl, ethyl, n-propyl or isopropyl, n-butyl, isobutyl or tert-butyl, pentyl, hexyl, heptyl or octyl group, all of which may also be branched, L is an ethoxy group or a chlorine or bromine atom and m is 0, 1 or 2, in the presence of a catalytic amount of one or more copper(I) and/or copper(II) compounds and of one (or more) silyl reagent(s) of the general formula III
R¹R²R³SiZ (III),
wherein
each of R¹, R² and R³ may be the same or different and is a straight-chained or branched-chained alkyl radical having from 1, or in the case of a branched chain from 3, to 10 carbon atoms, or an aryl radical that is optionally substituted by from 1 to 3 chlorine atoms or by from 1 to 3 straight-chained or branched-chained alkoxy or alkyl radicals having from 1, or in the case of a branched chain from 3, to 6 carbon atoms, and
Z is a chlorine, bromine or iodine atom, cyano, a perfluoroalkylsulphonyloxy radical [(CₙF₂ₙ₊₁SO₂O-) in which n is 1, 2, 3 or 4], the mesylate radical CH₃SO₂O- or the tosyl radical p-CH₃-C₆H₄-SO₂O-.

10. Process according to claim 9, characterised in that methylation is carried out with dimethylzinc, trimethylaluminium, dimethylaluminium chloride or dimethylaluminium ethoxide.

11. Process according to claim 9, characterised in that ethylation is carried out with triethylaluminium.

12. Process according to claim 9, 10 or 11, characterised in that alkylation is carried out in the presence of one or more compound(s) of the general formula I
CuX or CuX₂ (I)
wherein X represents a monovalent radical and is chlorine, bromine, iodine, cyano, a thienyl radical, a phenyl radical, an alkoxy or thioalkoxy radical wherein the alkyl radical contained therein has from 1 to 8 carbon atoms and is optionally branched and/or unsaturated, a substituted alkynyl radical R-C≡C- wherein R is a phenyl radical or an optionally branched C₁-C₈alkyl radical, or the radical of an inorganic or carboxylic acid, or, with the exception of the acetylacetonate ligand in the case of the bivalent copper, X is a bidentate complex ligand that co-ordinates by way of oxygen and/or nitrogen atoms,
and/or one or more compound(s) of the general formula II
Cu₂Y or CuY (II)
wherein Y represents a bivalent radical and is oxygen or sulphur.

13. Process according to claim 12, characterised in that alkylation is carried out in the presence of copper(I) and/or copper(II) chloride and/or bromide and/or copper(I) cyanide.

14. Process according to any one of the preceding claims 9 to 13, characterised in that alkylation is carried out in the presence of a total of from 0.1 to 10 mol % of the copper(I) and/or copper(II) compound, based on the α,β-unsaturated compound that is to be alkylated.

15. Process according to any one of claims 9 to 14, characterised in that alkylation is carried out in the presence of trimethylsilyl chloride, tert-butyldimethylsilyl chloride, tert-butyldiphenylsilyl chloride, trimethylsilyltriflate and/or trimethylsilyl cyanide as silyl reagent(s).

16. Process according to any one of the preceding claims 9 to 15, characterised in that alkylation is carried out in the presence of a total of from 10 to 1000 mol % of the silyl reagent, based on the compound that is to be alkylated.

17. Process according to any one of the preceding claims 9 to 16, characterised in that it is carried out in tetrahydrofuran, dioxane, dimethoxyethane or toluene as solvent

18. Process according to any one of the preceding claims 9 to 16, characterised in that it is carried out in ethyl acetate as solvent.

19. Process according to any one of the preceding claims 9 to 18, characterised in that it is carried out at a reaction temperature of from 0°C to 50°C.

20. Process according to any one of the preceding claims 9 to 19, characterised in that the α,β-unsaturated ketone is a 3-keto-1,4-diene steroid, a 3-keto-1-ene steroid, a 3-keto-4-ene steroid or a 17-acyl-16-ene steroid.

21. Process for the preparation of 1α-methylandrost-4-ene-3,17-dione as intermediate in the preparation of 1α-methyl-17β-hydroxy-5α-androstan-3-one (mesterolone), characterised in that androsta-1,4-diene-3,17-dione is methylated according to any one of claims 9 to 19.

22. Process for the preparation of a 3-acyloxy-1α-methyl-androsta-2,4-dien-17-one as intermediate in the preparation of 1-methyl-androsta-1,4-diene-3,17-dione (atamestane), characterised in that androsta-1,4-diene-3,17-dione is methylated according to any one of claims 9 to 19 and the enolate that is present in the reaction mixture after methylation is captured by a carboxylic acid anhydride or chloride of a straight- or branched-chained alkanecarboxylic acid having from 2 to 8 carbon atoms or of benzoic acid.

23. Process according to claim 22, characterised in that the enolate is captured by acetic anhydride or acetyl chloride.

## Revendications

1. Agent d'alkylation contenant un réactif à base d'aluminium Alk₃₋ₘA1Lₘ, où Alk est un groupe méthyle, éthyle, n- ou i-spropyle, n-, i- ou tert.-butyle, pentyle, hexyle, heptyle ou octyle, tous ces groupes pouvant aussi être ramifiés, L est un groupe éthoxy, un atome de chlore ou de brome et m vaut 0, 1 ou 2, comme source d'alkyle ou aussi le diméthyle de zinc comme source de méthyle, caractérisé en ce qu'il contient de plus des quantités catalytiques d'un ou plusieurs composés de cuivre(I) et/ou cuivre(II) et un (ou plusieurs) réactif(s) à base de silyle de formule générale III
R¹R²R³SiZ (III)
où
R¹, R² et R³ peuvent être identiques ou différents et représenter un radical alkyle linéaire ou ramifié avec 1, respectivement, dans le cas d'alkyle ramifié, avec 3, à 10 atomes de carbone, un radical aryle éventuellement substitué par 1 à 3 atomes de chlore ou par 1 à 3 radicaux alkyle, respectivement alcoxy linéaires ou ramifiés avec 1, respectivement, dans le cas de radical ramifié, avec 3, à 6 atomes de carbone ainsi que
Z représente un atome de chlore, de brome ou d'iode, le radical cyano, un radical perfluoroalkylsulfonyloxy [(CₙF₂ₙ₊₁SO₂O-), avec n = 1, 2, 3 ou 4], le radical mésylate CH₃SO₂O-, ou le radical tosyle p-CH₃-C₆H₄-SO₂O-.

2. Agent d'alkylation selon la revendication 1, caractérisé en qu'il contient le diméthylzinc, le triméthylaluminium, le chlorure de diméthylaluminium ou l'éthoxyde de diméthylaluminium comme source de méthyle.

3. Agent d'éthylation selon la revendication 1, caractérisé en ce qu'il contient le triéthylaluminium comme source d'éthyle.

4. Agent d'alkylation selon la revendication 1, 2 ou 3, caractérisé en ce qu'il contient comme composé de cuivre(I) et/ou de cuivre(II) un ou plusieurs composés de formule générale I
CuX ou CuX₂ (I),
où X est un radical monovalent et représente le chlore, le brome, l'iode, cyano, le groupe thiényle, phényle, un radical alcoxy, thioalcoxy où le radical alkyle contenu comporte 1 à 8 atomes de carbone et est éventuellement ramifié et/ou insaturé, représente un radical alcynyle R-C≡C-, R étant un radical phényle ou éventuellement un radical alkyle en C₁-C₈ ramifié, ou le radical d'un acide inorganique ou d'un acide carboxylique ou un ligand complexe bidenté de coordination par l'intermédiaire des atomes d'oxygène et/ou atomes d'azote, à l'exception du ligand acétonacétonate dans le cas du cuivre bivalent et/ou un ou plusieurs composés de formule générale (II)
Cu₂Y ou CuY (II)
où Y est un radical bivalent et représente l'oxygène ou le soufre.

5. Agent d'alkylation selon la revendication 4, caractérisé en ce qu'il contient le chlorure et/ou le bromure de cuivre(I) et/ou de cuivre(II) et/ou le cyanure de cuivre(I).

6. Agent d'alkylation selon l'une des revendications précédentes caractérisé en ce qu'il contient au total de 0,1 à 10% en moles de composé de cuivre(i) et/ou de cuivre(II) par rapport au composé à alkyler.

7. Agent d'alkylation selon l'une des revendications précédentes caractérisé en ce qu'il contient le chlorure de triméthylsilyle, le chlorure de tert.-butyl-diméthylsilyle, le chlorure de tert-butyldiphénylsilyle, le triflate de triméthylsilyle et/ou le cyanure de triméthylsilyle en tant que réactif(s) à base de silyle.

8. Agent d'alkylation selon l'une des revendications précédentes caractérisé en ce qu'il contient au total de 10 à 1000 % en moles du réactif à base de silyle par rapport au composé à alkyler.

9. Procédé pour l'addition d'un groupe alkyle alk (alk est un groupe méthyle, éthyle, n- ou i-propyle, n-, i- ou tert.-butyle, pentyle, hexyle, heptyle ou octyle, qui peuvent être tous ramifiés) sur la position 1,4 d'une cétone insaturée en α,β ou doublement insaturée en α,β ou d'un aldéhyde insaturé en α,β, caractérisé en ce qu'on alkyle la cétone insaturée en α,β ou doublement insaturée en α,β ou l'aldéhyde insaturé en α,β avec un réactif à base d'aluminium Alk₃₋ₘAlLₘ, où Alk est un groupe méthyle, éthyle, n- ou i-propyle, n-, i- ou tert.-butyle, pentyle, hexyle, heptyle ou octyle, tous ces groupes peuvent aussi être ramifiés, L est un groupe éthoxy, un atome de chlore ou de brome et m vaut 0, 1 ou 2,en présence d'une quantité catalytique d'un ou plusieurs composés de cuivre(I) et/ou cuivre(II) et d'un (ou plusieurs) réactif(s) à base de silyle de formule générale III
R¹R²R³SiZ (III)
où
R¹, R² et R³ pouvant être identiques ou différents et représenter un radical alkyle linéaire ou ramifié avec 1, respectivement, dans le cas d'alkyle ramifié, avec 3, à 10 atomes de carbone, un radical aryle éventuellement substitué par 1 à 3 atomes de chlore ou par 1 à 3 radicaux alcoxy, respectivement alkyle linéaires ou ramifiés avec 1, respectivement, dans le cas de radical ramifié, avec 3, à 6 atomes de carbone ainsi que
Z représente un atome de chlore, de brome ou d'iode, le radical cyano, un radical perfluoroalkylsulfonyloxy [(CₙF₂ₙ₊₁SO₂O-), avec n = 1, 2, 3 ou 4], le radical mésylate CH₃SO₂O-, ou le radical tosyle p-CH₃-C₆H₄-SO₂O-.

10. Procédé selon la revendication 9 caractérisé en ce qu'on effectue la méthylation par le diméthylzinc, le triméthylaluminium, le chlorure de diméthylaluminium ou le méthoxyde de diméthylaluminium.

11. Procédé selon la revendication 9, caractérisé en qu'on effectue l'éthylation par le triéthylaluminium.

12. Procédé selon la revendication 9, 10 ou 11 caractérisé en ce qu'on alkyle en présence d'un ou plusieurs composés de formule générale I
CuX ou CuX₂ (I)
où X est un radical monovalent et représente le chlore, le brome, l'iode, cyano, le groupe thiényle, phényle, un radical alcoxy, thioalcoxy, où le radical alkyle contenu dans ces radicaux comporte 1 à 18 atomes de carbone et est éventuellement ramifié et/ou insaturé, représente un radical alcynyle R-C≡C-, R étant un radical phényle ou un radical alkyle en C₁-C₈ éventuellement ramifié, ou le radical d'un acide inorganique ou d'un acide carboxylique ou un ligand complexe bidenté de coordination par l'intermédiaire des atomes d'oxygène et/ou d'azote, à l'exception du ligand acétylacétonate dans le cas du cuivre bivalent et/ou d'un ou plusieurs composés de formule générale II
Cu₂Y ou CuY (II)
où Y est un radical bivalent et représente l'oxygène ou le soufre.

13. Procédé selon la revendication 12, caractérisé en ce qu'on alkyle en présence de chlorure ou de bromure de cuivre(I) et/ou de cuivre(II) et/ou de cyanure de cuivre(I).

14. Procédé selon l'une des revendications précédentes 9 à 13, caractérisé en ce qu'on alkyle en présence d'un total de 0,1 à 10% en moles de composé de cuivre(I) et/ou de cuivre(II) par rapport au composé insaturé en α,β à alkyler.

15. Procédé selon l'une des revendications 9 à 14, caractérisé en ce qu'on alkyle en présence de chlorure de triméthylsilyle, de chlorure de tert-butyl-diméthylsilyle, de chlorure de tert-butyldiphénylsilyle, de triflate de triméthylsilyle et/ou de cyanure de triméthylsilyle comme réactif à base de silyle.

16. Procédé selon l'une des revendications précédentes 9 à 15, caractérisé en ce au'on alkyle en présence d'un total de 10 à 1000 % en moles du réactif à base de silyle par rapport au composé à alkyler.

17. Procédé selon l'une des revendications précédentes 9 à 16, caractérisé en ce qu'il est mis en oeuvre dans du tétrahydrofuranne, du dioxane, du diméthoxyéthane ou du toluène comme solvant.

18. Procédé selon l'une des revendications précédentes 9 à 16, caractérisé en qu'il est mis en oeuvre dans l'acétate d'éthyle comme solvant.

19. Procédé selon l'une des revendications 9 à 18 précédentes, caractérisé en ce qu'il est mis en oeuvre à une température de réaction de 0°C à 50°C.

20. Procédé selon l'une des revendications précédentes 9 à 19, caractérisé en ce que la cétone insaturée en α,β est un stéroïde 3-céto-1,4-diènique, un stéroïde 3-céto-1-énique, un stéroïde 3-céto-4-énique ou un stéroïde 17-acyl-16-énique.

21. Procédé pour la préparation de la 1α-méthylandrost-4-ène-3,17-dione comme produit intermédiaire pour la préparation de la 1α-méthyl-17β-hydroxy-5α-androstan-3-one (mestérolone), caractérisé en ce qu'on méthyle l'androsta-1,4-diéne-3,17-dione selon l'une des revendications 9 à 19.

22. Procédé de préparation d'une 3-acyloxy-1α-méthyl-androsta-2,4-diène-3,17-dione comme produit intermédiaire pour la préparation de la 1-méthyl-androsta-1,4-diène-3,17-dione (atemestane), caractérisé en ce qu'on méthyle l'androsta-1,4-diène-3,17-dione selon l'une des revendications 9 à 19 et que l'énolate présent après méthylation dans le mélange réactionnel est piégé par un anhydride ou chlorure d'acide carboxylique d'un acide acide alcanecarboxylique linéaire ou ramifié avec 2 à 8 atomes de carbone ou par l'acide benzoïque.

23. Procédé selon la revendication 22, caractérisé en ce que l'énolate est piégé par l'anhydride de l'acide acétique ou le chlorure d'acétyle.
